(19) **Europäisches Patentamt** / **European Patent Office** / **Office européen des brevets**

(11) **EP 4 614 506 A1**

(12) **EUROPEAN PATENT APPLICATION**

(43) Date of publication:
**10.09.2025 Bulletin 2025/37**

(21) Application number: **25161255.2**

(22) Date of filing: **03.03.2025**

(51) International Patent Classification (IPC):
**G16H 20/40** (2018.01)    **G16H 50/30** (2018.01)
**G16H 40/63** (2018.01)

(52) Cooperative Patent Classification (CPC):
**G16H 20/40; G16H 50/30;** G16H 40/63

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.03.2024 IT 202400004942**

(71) Applicant: **Technogym S.p.A.**
**47521 Cesena (FC) (IT)**

(72) Inventors:
- ZOFFOLI, Luca
  **47521 CESENA (IT)**
- TANA, Marcello
  **47521 CESENA (IT)**
- GABELLINI, Stefano
  **47521 CESENA (IT)**
- MONTANARI, Filippo
  **47521 CESENA (IT)**

(74) Representative: **De Santis, Giovanni et al**
**c/o Giambrocono & C. S.p.A.**
**Via Rosolino Pilo, 19/B**
**20129 Milano (IT)**

(54) **SYSTEM FOR ASSESSING AND IMPROVING THE FUNCTIONAL AGE AND/OR AN OVERALL INDEX INDICATIVE OF THE CURRENT STATE OF PSYCHOPHYSICAL WELL-BEING OF A USER**

(57)    System (100) for assessing and improving the functional age and/or an overall index indicative of the current state of psycho-physical well-being of a user, comprising:
- a check-up station (1) which comprises a local controller (10), a display (3) and a loudspeaker (5), and allows the user to connect to the system directly on the display or via an own portable electronic device (125);
- a remote controller (110) in communication with the check-up station and with one or more pre-selected exercise devices (130, 140).

At least one of the remote and local controllers is configured to present information and/or instructions for guiding a user in performing a plurality of tests each having a predefined time limit and being related to a physical or psychological domain of the user. The check-up station is configured to allow the execution of a first sub-set of tests, at least a second sub-set of tests being executable by one or more of the physical exercise devices.

The remote controller is also configured to calculate, based on the tests performed, an overall functional age value and/or said overall index and generate a new training program or modify/confirm a pre-existing training program.

FIG.1

**Description**

The present invention relates generally to the field of wellness.

In particular, the present invention relates to a system for assessing and improving the functional age and/or an overall index indicative current state of psycho-physical well-being of a user.

As it is well known, over the past decades, the so-called world of fitness and then the conceptually broader world of wellness have had a significant expansion.

Indeed, an increasingly large segment of the population has started to devote more time to physical exercise by frequenting gyms or fitness centres of various kinds more or less assiduously, even in old age.

Alongside the purely physical aspect, there has also been a growing focus on the psychological aspect, or what might be defined as 'brain fitness', and mental and emotional health are nowadays considered by users to be just as important as physical health.

Therefore, field professionals have gradually defined benchmarks and developed various methodologies, equipment and machinery suitable for measuring these parameters indicative of the physical and/or mental health status of users.

For example, a typical parameter used for the assessment of physical health is the so-called body mass index (BMI), internationally referred to by the acronym 'BMI' (Body Mass Index). However, this parameter does not provide a correct estimate of body composition on subjects with high muscle masses. For this purpose, an alternative technique is used, i.e. bioimpedance, which allows to indirectly detect the actual fat and muscle masses of a user.

Another parameter is the so-called $VO_2$max, which basically measures the ability of a user to consume oxygen and it is often correlated or measured together with the maximum heart rate.

Nowadays, there are various types of instruments and devices that measure one or the other of the most commonly used parameters in the industry.

In certain instances, on the basis of the measured parameter, usually a fitnessrelated parameter, some devices, such as the more modern smartwatches, provide also an indicative value of an age that in some cases is called biological age or functional age, or 'functional age' i.e. what would be, based on the measured parameter or domain, the age of a user in relation to their true anagraphic age reflecting the expected performance capacity of a user for a given age and sex.

However, known solutions, while calculating the functional age of a user, are susceptible to further improvement.

In particular, the value calculated for the presumed functional age, which usually represents a merely indicative value and an end in itself, is calculated on a rather limited and partial data base, and therefore it may not be particularly precise and reliable.

Moreover, this assessment often remains an extemporaneous indication without suitable follow-up for the purpose of its improvement.

Therefore, the present invention is intended to provide an improved or at least alternative solution compared to the solutions of the prior art, and in particular one that allows to provide users not only an assessment of their functional age, and/or an overall index indicative of their current psycho-physical state, that is as reliable as possible, but also one that allows its improvement in a guided and monitored way over time.

This purpose, and others that may result from the description below, is achieved by a system for the assessment and improvement of functional age and/or an overall index indicative of the current state of psycho-physical well-being of a user whose characteristics are defined in claim 1.

Particular embodiments are the subject of dependent claims, the content of which is intended to be an integral part of the present description.

Further features and advantages of the invention will appear from the following detailed description, carried out by way of non-limiting example only, with reference to the accompanying drawings, in which

Figure 1 is a block diagram schematically illustrating a system for assessing and improving functional age and/or an overall index indicative of the current state of psycho-physical well-being of a user according to the invention;
Figures 2-4 are perspective views illustrating possible embodiments of a check-up station that can be used in the system of Figure 1;
Figures 5-9 schematically illustrate some of the test performance steps on the check-up station of Figures 2-4;
Figure 10 illustrates a possible embodiment of a graphical user interface of the check-up station that can be used in the system of Figure 1.

It should be noted that in the detailed description that follows, in order to clearly and concisely describe the present invention, the drawings may not necessarily be to scale and some features of the description may be shown in a somewhat schematic form.

Furthermore, when the term "configured", or "adapted", or "shaped", or "set", or any similar term is used herein, referring to any component as a whole, or to any part of a component or to a combination of components, it should be intended that it means and includes the structure and/or configuration and/or shape and/or positioning correspondingly .

In particular, when these terms refer to electronic hardware or software, they are to be understood as including electronic circuits or parts of electronic circuits, as well as software/firmware, such as algorithms, routines and programs in general, running and/or resident in any storage element.

Finally, in the following description and claims, the ordinal numerals first, second, et cetera, will be used for the sake of illustrative clarity and in any way should they be intended as limiting for any reason whatsoever; in particular, the indication of e.g. a "second test", or a "second device", does not necessarily imply the presence or strict requirement that there is a further "a first or third test", or "a first or third device", and vice versa, unless such presence is clearly evident for the correct functioning of the described embodiments, nor that the order must be that exactly in the described sequence (first, second, third, etc.) with reference to the illustrative described embodiments.

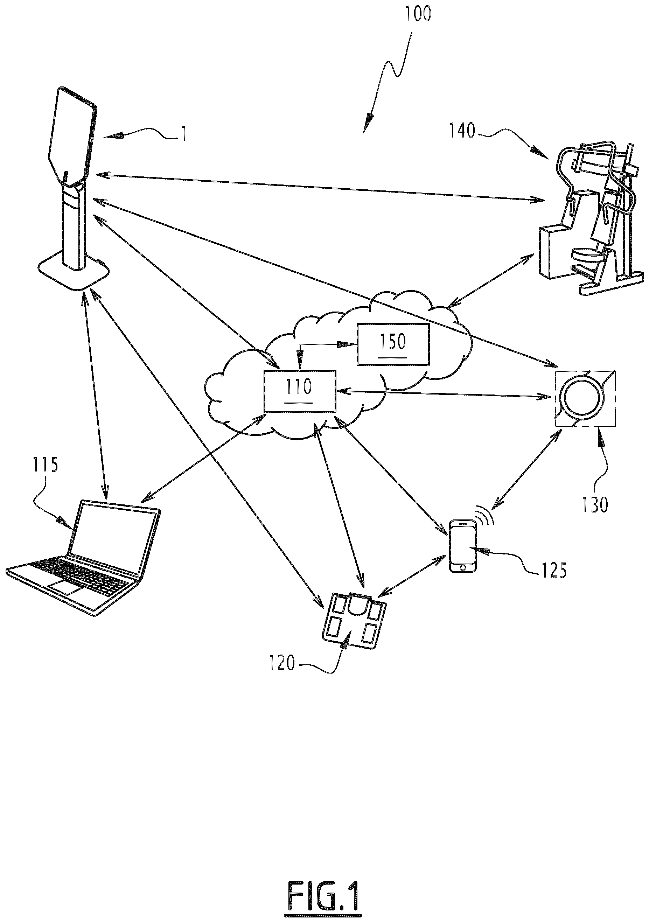

Figure 1 schematically illustrates a system for assessing and improving functional age and/or equivalently of an overall index indicative of the current state of psycho-physical well-being of a user according to the invention, indicated by the global reference number 100, comprising at least one check-up station 1, and an electronic processing unit or remote controller 110 (hereinafter referred to as remote controller 110).

The overall index can be, for example, a value expressed on a predefined scale, e.g. a percentile, where higher values correspond to a better psycho-physical condition.

In the following description, particular reference will be made to the functional age value for the sake of descriptive simplicity.

The remote controller 110 is suitable to be placed in communication with at least the check-up station 1, and with one or more predefined physical exercise devices 130, 140 whose identifying data, related in particular to their type, technical characteristics and physical location, i.e. the site(s) where they are installed, are pre-registered in at least one data recording unit 150, e.g. one or more servers, databases or memories, accessible from the remote controller 110 itself.

The at least one recording unit 150 may be part of or associated with the remote controller 110 and one or more algorithms may be stored thereon, whose execution allows to realize the expected functionality for at least the remote controller 110 within the system 100 according to the present invention, as will be described in more detail below.

In a possible embodiment, the remote controller 110 is cloud-based and thus may comprise or consist of, for example, one or more servers in a mode or based on so-called cloud computing.

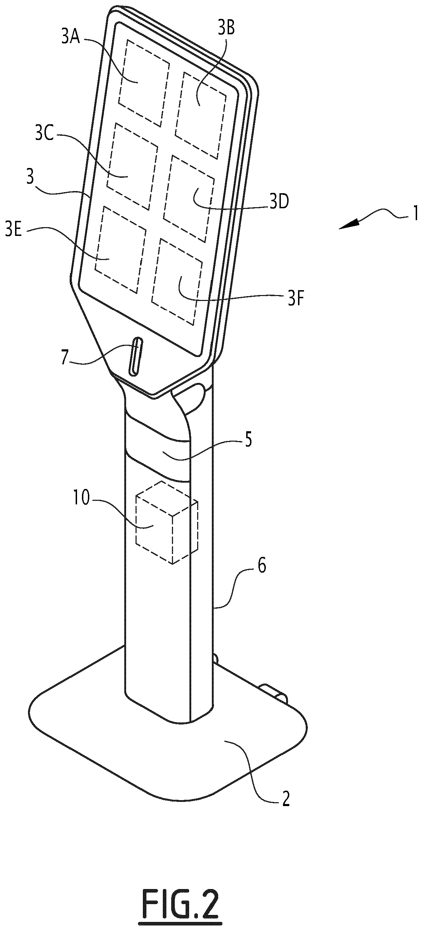

Figures 2 to 4 are perspective views illustrating possible embodiments of a check-up station 1 used in system 100 according to the present invention.

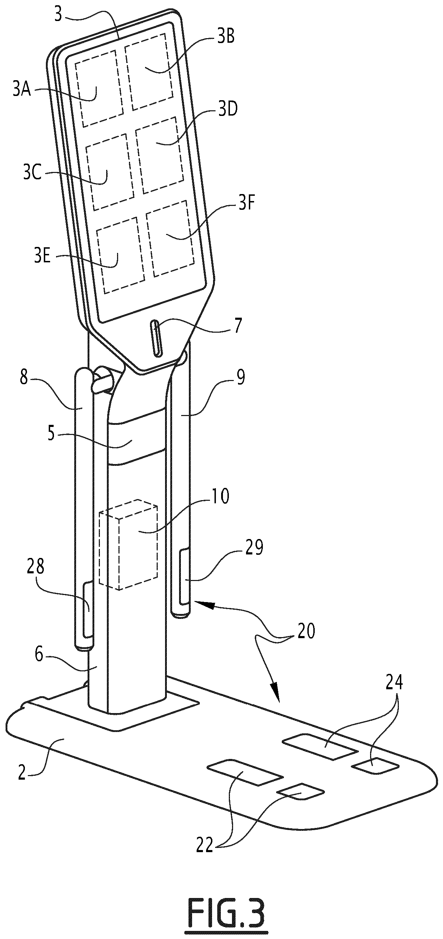

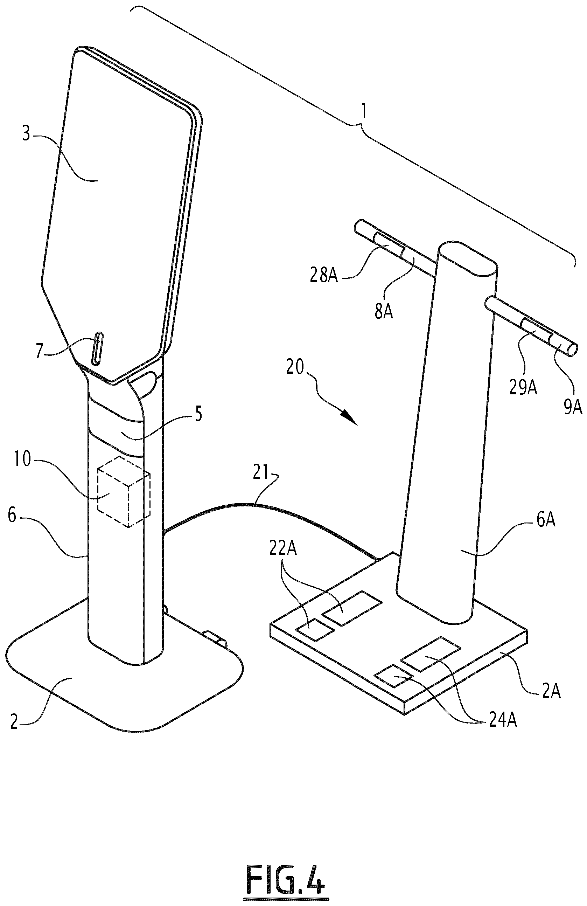

Here, the definition of check-up station 1 is to be understood as including either an equipment realised in a single structure (see e.g. Figures 2 and 3) that integrates the various components/constituent parts, or equipment realised by means of two or more separate and interconnected parts/devices (see e.g. Figure 4), and in this latter case it may be considered for example as constituted of two or more check-up substations.

As illustrated, the check-up station 1 comprises at least one of a display 3 and a loudspeaker 5, preferably both, and a local controller 10, i.e., a controller integrated into the station 1 itself, and it is configured to allow the user to connect to the system 100, and in particular to the remote controller 110, directly on the display 3 itself, for example via a login interface appearing on the screen of the display 3, or via a portable electronic device 125 of the user himself or herself, for example a smartphone.

On such a portable electronic device 125, the user can download a dedicated App that is part of or allows interaction with the system 100.

For example, a user who desires to be tested can interact or connect directly to the station 1 and to the remote controller 110 operationally connected to station 1, and possibly access their own data if they already existing or generate ex-novo their own profile with their own relevant data, e.g. through their smartphone 125 reading a QR code on display 3 or simply by entering their own identification data, e.g. username and password.

For example, each user, when generating their profile or when updating it, can enter their personal data, anthropometric data, etc., e.g. age, sex, weight, height, limb size, etc.

User data can also be downloaded e.g. from a PC/tablet/mobile phone or entered manually by the user himself or herself or by a trainer.

User data, including e.g. their personal record of programs, trainings, fitness status et cetera, can be recorded remotely, e.g. in the cloud, e.g. in the recording unit 150, and/or even locally in station 1 itself.

The local controller 10 may be constituted of or comprise an electronic control unit including, for example, a processor, one or more software modules or algorithms which, when executed by the processor, allow the execution of the various functionalities expected for the local controller 10, one or more communication modules for data transceiver with the external environment, for example with the remote controller 110 and with other devices, such as the devices illustrated in Figure 1.

Local controller 10 may also include, for example, at least one or more local data storage units.

Usefully, in the system 100 according to the invention, at least one of said remote controller 110 and said local controller 10, preferably the remote controller 110, is configured so that, through at least one of the display 3, the loudspeaker 5, and the portable electronic device 125 of the user, information and/or instructions are presented to the user for guiding the user in performing a plurality of different tests, each test having a predefined time deadline and being related to a physical or psychic domain of the user to be tested.

In particular, the check-up station 1 is configured to allow the performance of a first subgroup of tests of the plurality of tests, and at least a second subgroup of tests of the plurality of tests can be performed by one or more pre-selected physical exercise devices 130, 140.

Usefully, the system 100 according to the invention is configured to test a user by performing at least two tests belonging to the second subgroup, i.e. a first test suitable for assessing the current level of muscular strength, and a second cardio test suitable for assessing the current cardiovascular status of the user, and at least two other tests belonging to the first subgroup to be performed by means of the check-up station 1, i.e. a test suitable for assessing the current level of mobility of the user, and another test suitable for assessing the current level of balance ability of the user himself or herself.

In addition, the system 100 is usefully configured to test a user in at least two further domains, in particular that of current body mass composition and that of current cognitive ability; these further two tests, as will become more detailed from the following description, can be performed using check-up station 1 directly or external or associated devices.

In particular, the display 3 is equipped with a graphic interface that presents to the user one or more windows 3A, 3B, 3C, 3D, 3E, 3F for selecting and guiding the execution of one or more tests of interest.

Clearly, both the shape of display 3, as well as the graphic display of windows 3A, 3B, 3C, 3D, 3E, 3F, can take on any other configuration than that illustrated in Figures 2 to 4.

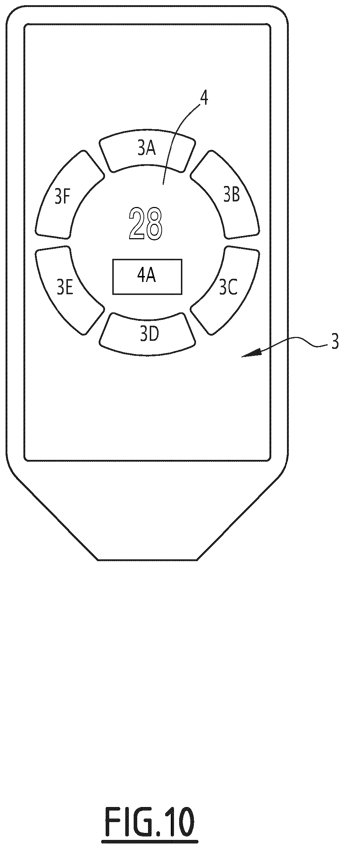

For example, in a possible embodiment illustrated in figure 10, windows 3A, 3B, 3C, 3D, 3E, 3F are arranged in a circular or flower petals pattern, and the inner area delimited by windows 3A, 3B, 3C, 3D, 3E, 3F, constitutes an additional window 4 on which the user can interact.

In addition, within the area of window 4, one or more further windows can be defined, e.g. a 4A start window that the user can tap to begin navigation within system 100.

In addition, the check-up station 1 comprises a camera 7 configured to capture and send to the remote controller 110 and/or the local controller 10 one or more images of the user when he or she is performing the one or more tests of the first subgroup, i.e. those to be performed directly at the check-up station 1.

For example, in the embodiments illustrated in Figures 2 to 4, the check-up station 1 comprises at least one resting base 2 and a support 6 rising from the resting base 2.

For example, the loudspeaker 5 is arranged along the support 6.

For example, the display 3 is arranged at the opposite end of the support 6 relative to the from resting base 2.

For example, the camera 7 is arranged at the base of display 3.

The camera 7 can for example be a 2D digital camera, a 3D digital camera, an RGB camera, an RGB-D camera, a video camera, a depth camera, a TOF (Time-Of-Flight) camera, a webcam, a thermal imaging camera, et cetera.

The check-up station 1 may be installed at the same site, e.g. a training centre, e.g. a gymnasium, where one or more of the pre-selected physical exercise devices 130, 140 are installed, or it may be installed at a site remote from those devices 130, 140, and the same pre-selected devices 130, 140 may be installed at the same site, or at several different sites.

The local controller 10 is further configured to collect and send to the remote controller 110 data collected during the execution of each test of the first subgroup of tests performed by the user at check-up station 1, or on an associated device.

Conveniently, in the system 100 according to the invention, the remote controller 110 is further configured to:

- calculate, on the basis of the received data, related to the performed tests, a specific functional age value and/or an equivalent domain-specific reference index for each domain whose corresponding test has been performed;
- calculate an overall functional age value and/or said overall user index on the basis of the functional age values and/or reference indexes specifically calculated for the tests performed; and
- in function of at least one parameter indicative of the tested user (e.g. age and/or gender), and/or at least one specific functional age value or calculated reference index, and/or the overall functional age value or calculated overall index, generate a new training program or modify/confirm a pre-existing training program by making it accessible to the user via at least one of the display 3 and the portable electronic device 125.

For example, a code, e.g. a QR code, is displayed on display 3 of check-up station 1, and, when scanned with the portable device 125, allows the user access to their training content via the App.

Alternatively, the training program may already be available on a user account and can be retrieved through the App.

This program is, for example, automatically generated/edited on the cloud according to predefined logics.

For example, the controller 110 may define at least in part and/or present to the user one or more training sessions/exercises to be performed, depending on the overall calculated functional age (and/or index) value, or one or more training sessions/exercises specifically aimed at separately improving one or more of the calculated functional age (and/or respective index) values for each tested domain or aspect, including exercises/workouts of a physical type aimed at improving the tested cognitive ability.

A training program can also be defined based on the result of a single test and/or subtest. For example, if a neck mobility test is performed, if the measured angle is below an 'ideal' threshold, the suggested training program will include exercises to increase neck mobility. Similarly, if high oscillations of the torso or other body part were measured in the balance test, the suggested training program will include exercises to increase balance skills. Thus, if the tests/subtests of the various domains show 'frailties' compared to 'ideal' values, the suggested training program will usefully include exercises to improve and compensate for these specific frailties.

Each specific reference index for each tested domain can also be a value expressed on a corresponding predefined scale, e.g. a percentile, whereby higher values correspond to a better state for the user with regard to the tested domain.

In the following description, particular reference will be made to the specific functional age value for the sake of descriptive simplicity.

Data related to performed tests can be received by the remote controller 110 from the check-up station 1, and/or by one or more of the physical exercise devices 130, 140, and/or by the portable electronic device 125, and/or by further devices 115, 120 by means of which the plurality of tests are performed, as described in more detail below.

In a possible embodiment, the remote controller 110 is further configured to display on at least one of said display 3 and portable electronic device 125 of the user, the specific functional age values (and/or reference indices) calculated for each tested domain and the overall functional age value.

The overall functional age value can be calculated, for example, as an average of the various functional age values specific for each of the domains or aspects that are tested and taken into account, and it can be displayed in window 4 of display 3 as illustrated in figure 10.

In a possible embodiment, at least one between the local controller 10 and the remote controller 110 (preferably at least the latter), is configured to guide the user in performing the tests and each of the test steps according to a predetermined sequence.

For example, when the user logs in, remote controller 110 allows to display step by step the instructions to be followed, indicating in sequence the first test to be performed and the sections relating to it, then the second test, et cetera.

The status of running tests can be further emphasised by means of the graphics in display 3, e.g. by suitably illuminating the various windows for each text with different colours/intensities, e.g. depending on whether a test is yet to be started, is next to be performed, is partially performed, or is completed.

Clearly, the system 100 is configured in such a way that, if a user does not wish to follow the predefined sequence proposed by remote controller 100 (and/or local controller 10), the user has the option of proceeding according to their own choices, for example by skipping one test proposed in the sequence and performing another one.

In a possible embodiment, the remote controller 110 is further configured in such a way as to:

- check whether each test of the plurality of tests has been performed and is still temporally valid; and if not,
- invite the user to perform each test that has not yet been performed and/or repeat a temporally expired test.

In particular, according to one possible embodiment, the remote controller 110 is further configured in order to calculate an overall functional age value (and/or said overall index) of the user based on and only when all tests of the plurality of tests to be performed have been performed and are still temporally valid.

In a possible embodiment of the system 100 according to the invention, in response to a user selecting, for example on the display 3, a test to be performed belonging to the second subgroup of tests, i.e. a test configured to be performed elsewhere than at the check-up station 1, the remote controller 110 is further configured to:

- send to the user, directly on the display 3 or on the portable electronic device 125 of the user, one or more instructions identifying at least one device among the one or more of said physical exercise devices 130, 140 pre-selected with which to perform said test belonging to the second test subgroup.

In particular, according to a possible embodiment, the remote controller 110 is further configured in order to send on at least one of said at least one identified device 130, 140 and portable electronic device 125 one or more instructions suitable for guiding the user in performing said test belonging to the second test subgroup.

Usefully, the remote controller 110 is further configured in order to remotely configure said at least one device 130, 140 identified from those preselected on which the user shall perform the test chosen by the user belonging to the second test subgroup.

In particular, this remote configuration can be performed taking into account one or more data and/or parameters relating to the user himself or herself.

For example, if data is already pre-registered in the profile of the user, e.g. anthropometric data relating to one or more of their total height, total length of his upper and/or lower limbs, and/or parts thereof, torso size, etc., the remote controller 110 can configure the one or more selected device(s) 140 on the basis of one or more of such data (and also others such as the age and gender of the user.

Alternatively, in a possible embodiment, at least one of the local controller 10 and the remote controller 110, preferably the latter, is configured in order to guide the user, through the check-up station 1 acting as an interface, in the execution of one or more actions allowing to obtain some anthropometric data relevant to the remote configuration of the devices 140.

For example, the remote controller 110 may send instructions to the user, via the display 3 (and/or via the loudspeaker 5), to assume a certain position in front of the display; the camera 7 captures one or more corresponding images which are sent to the controller 10 of the station 1 and/or the remote controller 110.

For example, in one possible embodiment, the local controller 10, through the use of an image/movement analysis module or algorithm, analyses the one or more captured images, e.g. by calculating the total height of the user, the length of his arms and legs, or their parts, e.g. the length between shoulder and elbow and between elbow and hand, between pelvis and knee and between knee and ankle, etc.

Alternatively, the captured images may be sent directly to the remote controller 110, which analyses them in a similar way as described above for the local controller 10 to calculate the corresponding measurements.

The data obtained is then saved in the profile of the user (e.g. in the unit 150) and used to configure the one or more selected devices 130, 140. For example, when a user selects the window 3A relating to the execution of a first test of the second group of tests suitable for assessing the current level of muscular strength, the remote controller 110 selects one or more devices 140 from those pre-selected by sending to the user directly on the portable electronic device 125 and/or on the display 3 one or more instructions identifying each device identified to perform such test.

In addition or alternatively, such instructions (or others useful for the purpose of performing this test) may be sound instructions emitted via loudspeaker 5.

The remote controller 110 may identify and suggest performing the strength test by using a corresponding device 140, e.g. various types of presses or traction machines, for each relevant muscle group, and possibly configure them remotely for this purpose.

Once the user has gone to the indicated device(s) 140, he or she can make the identification, e.g. via their portable electronic device 125, and can perform the test.

During the performance, each device 140 used by the user transmits data directly to the remote controller 110 which, on the basis of the data received and appropriately processed, calculates a functional age value (and/or the respective reference index) related to the muscle force domain and an overall functional age value of the user also on the basis of this related muscle force value.

For example, the current strength level of the user can be calculated on the basis of the maximum strength (one-repetition maximum, commonly abbreviated to 1-RM), i.e. the maximum weight that can be lifted by a user for one single repetition in a given exercise, and it is indicative of their dynamic maximum strength.

This measured parameter can be compared by the remote controller 110 with pre-recorded data (or datasets), e.g. related to scientific investigations/studies previously carried out and/or from tests/workouts previously performed by the same user, and eventually calculate the sixth functional age value of the user on the basis of this comparison.

For example, the construction of the dataset can be carried out as follows:

each time the user performs a new strength training session on exercise machines that allow automatic tracking of the training results or storing the results of the performed training (e.g. manually by the user as instructed), the 1RM is estimated for each set performed using the formula known from the literature:

$$1RM\,(kg)\;=\;isoWeight\,(kg)\cdot \begin{cases} \dfrac{36}{37-isoRep} & \forall\, isoRep\,<\,9 \\[2mm] \dfrac{1}{0.522+0.419\cdot e^{0.055\cdot isoRep}} & \forall\, isoRep\,\geq\,9 \end{cases}$$

The used devices 140 may include, for example, a chest press, a leg press, and a vertical traction machine.

Once the 1RM value (last saved value) has been calculated and saved, system 100 controller 110 checks whether the last saved value is better than the most recent recorded value, within a predefined time window.

Following this check, the highest value is selected as the 'valid' value.

Each time the 1RM value is updated, it is then converted via experimental coefficients into the equivalent 1RM value on a reference exercise and/or reference equipment.

For example, the user performs a series of exercises on a flat bench and he or she records on the cloud (i.e. in the remote controller 110 or an associated data storage unit), the series as 'performed' with the assigned load.

The remote controller 110 calculates the 1RM for the exercise performed on the flat bench using the formula indicated above and converts this 1RM value on the flat bench into an equivalent 1RM value on a reference chest press using a conversion coefficient. Conversion coefficients can be derived through 'Big Data' analysis and each coefficient is gender/age specific.

The same operations can be carried out for the exercises performed with the other devices/machinery used, and a plurality of 1RM values is derived, e.g. three, one for each of the three types of machineries/devices used above.

Each of these three reference 1RM values is normalised for example as a function of the BMI of the user, and a polynomial function of variable degree can be defined for each 1RM value-user gender combination.

In this way, a reference dataset (e.g. static with periodic updating) can be constructed so that for each normalised reference 1RM, the expected value as a function of gender and age is available.

In the test phase, if the specific user does not have any 1RM value already pre-recorded, it is sufficient for him/her to perform an exercise to calculate the 1RM as stated above. In other cases, each time the user performs an exercise and this results in a higher 1RM value respect to the saved one, this expected value is compared by gender and by the corresponding reference exercise.

The functional age that is closest to the calculated 1RM value is then assigned and if two possible values are found, the one closest to the actual age is assigned.

The functional age value is assigned in this way for each of the calculated 1RM values (three in the example above), and the overall functional age related to muscle strength is derived by averaging these functional ages, e.g. between the three assigned functional ages.

Similarly, when a user selects, for example, the window 3B related to the performance of a test of the second group of tests adapted to assess a cardiovascular condition of the user, the remote controller 110 selects one or more cardio devices 130 external to the station 1 from those pre-selected, sending to the user directly on the portable electronic device 125 and/or on the display 3 one or more instructions identifying each device 130 identified to perform such test.

Such an external device 130 may be any instrument suitable for measuring a parameter related to the cardiovascular status of the user, and may be operatively connected to the remote controller 110 of the system 1 to communicate data related to the test performed or being performed. Examples of such external devices 130 are treadmills, bikes, ellipticals etc., for example provided with or connected to a heart rate monitor.

Once again, once the user has gone to the indicated cardio device(s) 130, he or she can make the identification, e.g. via their portable electronic device 125, and can perform the cardio test.

The remote controller 130 can remotely configure also such devices 130.

During performance, each cardio 130 device used by the user transmits data directly to the remote controller 110 which, on the basis of the received and appropriately processed data, calculates a specific functional age value (and/or the respective reference index) related to the cardiovascular domain, and an overall functional age value of the user also on the basis of this value related to the cardiovascular status.

For example, this specific functional age value related to the cardiovascular domain can be calculated on the basis of a parameter indicative of the aerobic capacity of the tested user, e.g. his $VO_2max$.

Also in this case, what is measured can be compared by the remote controller 110 with pre-recorded data, e.g. related to pre-executed scientific investigations/studies, and finally calculate the corresponding functional age value of the user on the basis of this comparison.

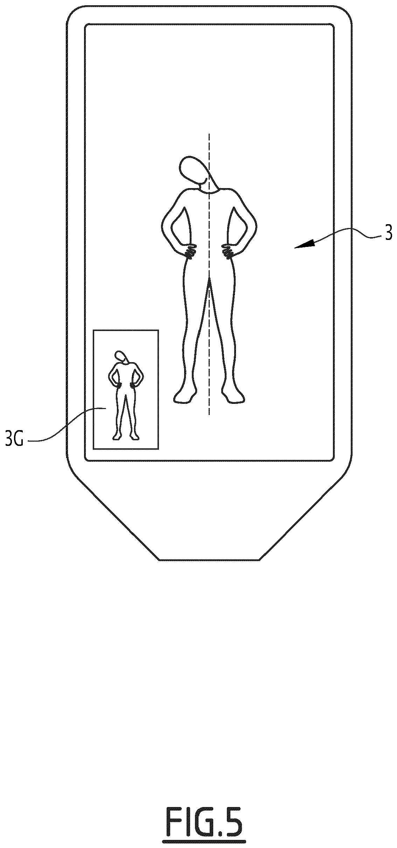

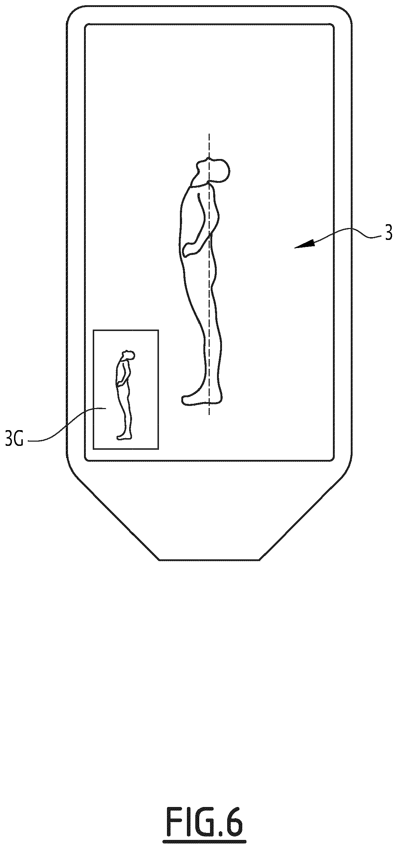

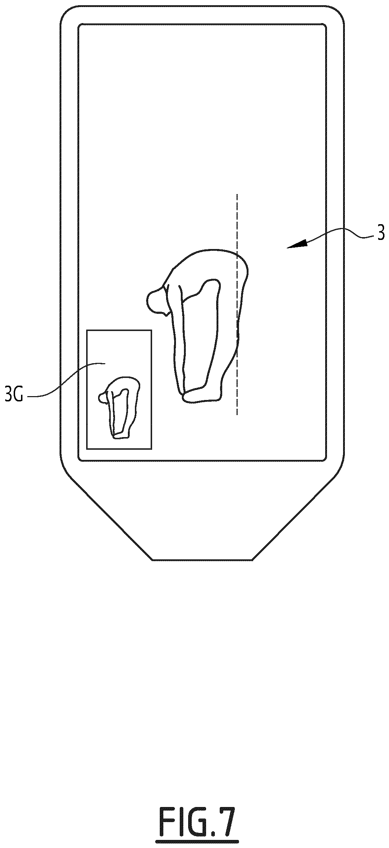

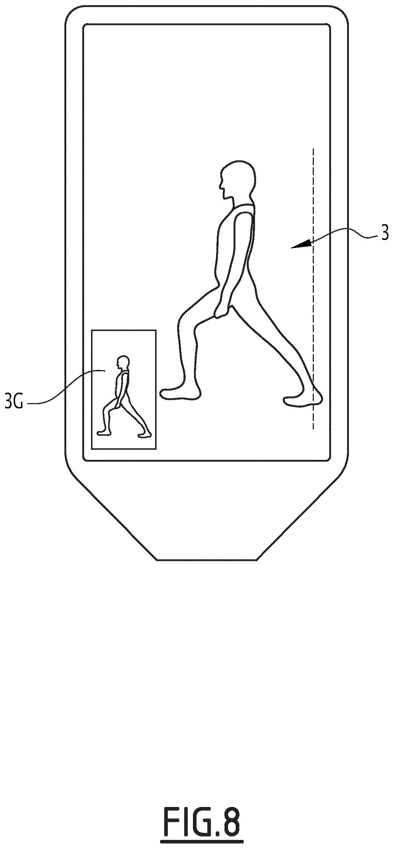

Instead, when a user selects on the display 3 a test to be performed by the station 1 itself, for example the window 3C related to the performance of a test adapted to assess a current level of mobility or flexibility of at least one part of the body of the user, the controller 10 or the remote controller 110 causes the display 3 to show a series of instructions to perform certain movements, e.g. bend the head to the right (and/or left) as shown in figure 5, extend the neck backwards as far as possible (figure 6), bend forward downwards and hold the position (figure 7), perform a lunge with one leg forward (figure 8).

In addition or alternatively, such instructions (or others useful for the purpose of performing this test) may be sound instructions emitted via the loudspeaker 5.

While performing the instructions received, the camera 7 captures one or more corresponding images which are sent to the controller 10 of the station 1, and/or to the remote controller 110.

For example, in one possible embodiment, the local controller 10, through the use of an image/movement analysis module or algorithm, analyses the one or more captured images, e.g. by calculating the angles of inclination of the body parts affected by the exercise and reached by the user being tested.

For example, during the performance of the test, several frames per second are detected and for each frame points of interest, so-called nodes or key points, are extracted, e.g. the body of the user is schematised with a segmented structure connected by nodes at the joints or other important points. The structure may be more or less dense with nodes. The extracted key points are compared with "ideal" reference key points, and the collected data related to key points and to respective evaluations, for all frames, are sent to the remote controller 110.

Alternatively, the images can be sent directly to the remote controller 110 which analyses them in the same way as described above for the local controller 10.

In both cases, these data obtained during the test are compared by the remote controller 110 with pre-recorded data, e.g. related to pre-performed scientific investigations/studies, and eventually a specific functional age value of the user is calculated based on the results of the mobility test performed.

Similarly, through the check-up station 1, the user can select the 3D window related to the execution of a test adapted to assess a current level of the balancing ability of the user.

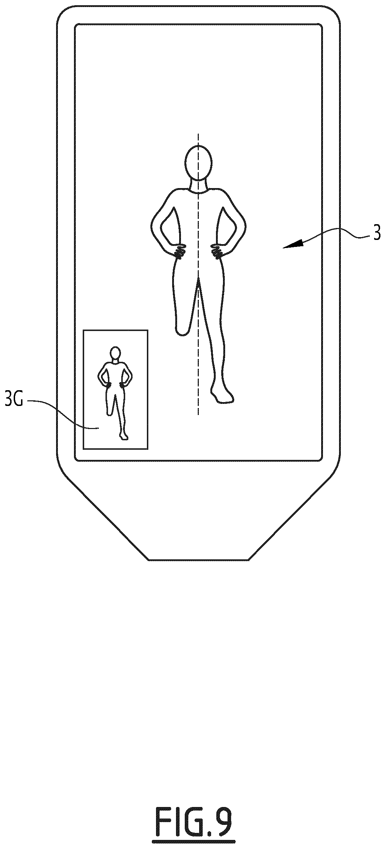

In this case, the controller 10 or the remote controller 110 causes the display 3 to show (and/or emits via the loudspeaker 5) a series of instructions to perform certain movements, e.g. to lift one foot off the ground (e.g. first the left and then the right) by folding it towards the back of the thigh and to hold the position for as long as possible or in any case for a certain time interval, as for example illustrated in figure 9.

Again, in one possible embodiment, the controller 10, through the use of an image/movement analysis module or algorithm, which may be the same as that used in the mobility test or a different module, analyses the one or more captured images, e.g. by calculating the length of time the user being tested held the required position.

In addition or alternatively, other parameters such as the swaying of the torso, of the pelvis and/or of the legs, during a predetermined time interval, are measured and the current level of the balance ability of the user is determined on the basis of these parameters.

Data obtained during the test are sent to remote controller 110, which compares it with corresponding pre-recorded data, e.g. related to pre-executed scientific investigations/studies, and eventually calculates a specific functional age value of the user based on the results of the balance test performed.

When performing tests by means of the check-up station 1, the instructions emitted on the display 3 (and/or via the loudspeaker 5) may be accompanied by the projection of a reference image on a window 3G of the display 3, as illustrated in Figures 5 to 9, and/or they may be orally emitted via the loudspeaker 5.

The remote controller 110 can then proceed and calculate the overall functional age value (and/or said overall index) also on the basis of these specific values related to the tested domains mentioned above.

Usefully, as previously indicated, the system 100 according to the invention is configured to assess and improve the overall functional age (and/or said overall index) of a user also on the basis of tests that can be performed, depending on requirements and application configurations, either on devices external to the check-up station 1, or using the check-up station 1 itself.

For example, in a possible embodiment, at least one between the local controller 10 and the remote controller 110 (preferably the latter) is further configured to cause on the display 3 the display of another window 3E relating to the selection of a test suitable of assessing a functional age (and/or respective reference index) specifically related to the level of current cognitive ability of the user, and a further window 3F related to the selection of a test suitable for evaluating a functional age (and/or respective reference index) specifically related to the current body mass composition of the user.

For example, if the user selects the window 3E related to the execution of a test to assess the current level of cognitive ability of the user, in a possible embodiment, at least one between the local controller 10 and the remote controller 110 causes the display on the display 3 of a series of instructions to perform, for example, one or more subtests/exercises known in themselves and for this reason not described therein in detail, for example to assess, following a sensory stimulus (e.g. visual), the current speed/accuracy of an expected response, and/or various types of memory, and/or concentration/attention ability, et cetera.

In this case, the check-up station 1 acts as a direct interface for the user, who can perform the exercises and/or issued instructions directly on station 1 itself.

In addition or alternatively, such instructions (or others useful in order to also perform this test) can be audio instructions emitted via the loudspeaker 5.

Alternatively, the system 100 comprises a further device 115 external to the station, which is capable of being placed in communication with and sending to the remote controller 110 data indicative of at least one test, performed or being performed by the user, to assess a level of cognitive ability of the user himself or herself.

The device 115 can be any device suitable for the purpose, e.g. a computer, tablet, laptop PC, smartphone, et cetera, and it can be pre-configured to perform such a test, or it can also be remotely configured by the remote controller 100 which can, for example, transmit the subtests/exercises to be performed.

In both cases, the remote controller 110 is further configured to receive data from the station 1 or the device 115 concerning the test being performed or executed.

The results of the cognitive test, expressed e.g. in terms of score, are compared also in this case by the controller 110 with pre-recorded data, e.g. related to pre-performed scientific investigations/studies, finally going on to calculate a value of the functional age of the user (and/or the corresponding reference index) on the basis of the results of the cognitive ability test(s) performed, and a value overall of the functional age of the user (and/or said overall index) also on the basis of this cognitive domain.

In a possible embodiment of the system 100 according to the invention, the check-up station 1 incorporates means for measuring at least one parameter indicative of the body mass composition of said user, or comprises a device (indicated in Figures 3 and 4 by reference number 20) configured to measure at least one parameter indicative of the body mass composition of said user.

For example, such means or device may be of the impedance type, e.g. to measure muscle mass and/or lean mass.

In particular, in a possible embodiment, as for example illustrated in Figure 3, the check-up station 1 incorporates, as means for measuring at least one parameter indicative of the composition of the body mass of the user, a first arm 8 and a second arm 9 extending from opposite sides to each other with respect to the support.

Furthermore, said means comprise at least one pair of electrodes 22, 24 arranged on the resting base 2, on which the user stands during the measurement, and a first electrode 28 and a second electrode 29 which are arranged along said first arm 8 and second arm 9, respectively, and which are suitable to be grasped by the user for the measurement of said parameter.

In the form of embodiment illustrated in figure 4, the check-up station 1 is instead formed by at least two sub-units connected to each other and comprises a main body substantially reproducing the form of embodiment of figure 2, and a device 20 for measuring at least one parameter indicative of the body mass composition of the user, which is formed by a second body operatively connected to the main body of the station 1, e.g. wired via one or more cables 21.

In this case, device 20 includes:

- an additional resting base 2A;
- an additional support 6A rising from the additional resting base 2A;
  a third arm 8A and a fourth arm 9A extending from opposite sides of the additional support 6A;
- at least one pair of electrodes 22A, 24A which are placed on the additional resting base 2A and on which the user stands during the measurement of the first parameter; and
- a third electrode 28A and a fourth electrode 29A which are arranged along the third arm 8A and the fourth arm 9A, respectively, and are suitable to be grasped by the user during the measurement of the first parameter.

In practice, if the user selects the window 3E related to the performance of a test suitable for testing their current body composition, either via the means directly incorporated in the station 1 or via the device 20, the user is subjected to an impedance test whose data is used by the remote controller 110 to calculate a functional age value specifically related to the current body composition (and/or said reference index), and an overall functional age value of the user (and/or said overall index) also based on said specific domain.

For example, also in this case such data obtained during the impedance test are compared by the remote controller 110 with pre-recorded data, e.g. from pre-executed scientific investigations/studies, ultimately calculating a specific functional age value of the user (and/or the relevant reference index) on the basis of the results of the impedance test performed.

For example, the functional age value related to the current body composition could be determined from the registry age, decreased/increased by a certain amount depending on the comparison of the test results with ideal values of parameters such as fat mass, lean mass, BMI.

In the various embodiments illustrated in Figures 2 to 4, the station 1 can also include one or more load cells, integrated for example into the base, to measure the weight of a user; this data can be used by the remote controller 110 to calculate the various functional ages or the overall functional age.

Alternatively, the remote controller 110 can be configured to:

- receive as input at least one parameter measured by a device external to station 1, indicative of the body mass composition of said user;
- calculate a functional age value of a user (and/or the respective reference index) specifically related to the current body mass composition on the basis of said at least one received parameter; and subsequently
- calculate an overall functional age value of the user (and/or said overall index) also taking into account the specific functional age value (and/or respective reference index) related to this tested domain.

Such an external device, indicated schematically in figure 1 by the reference number 120, can be any instrument suitable for measuring the composition of the body mass of a user, e.g. an impedance balance.

In this case, the remote controller 110 and/or the station 1 may receive the measured parameter directly from the device 120, if such a device is equipped with communication/connection capabilities, or from a device through which the user can input and send the measured value, e.g., the portable electronic device 125, e.g., their mobile phone.

In practice, it has been found that the system 100 for assessing and improving the functional age (and/or said overall index) of a user according to the invention fulfils the intended purpose in that it allows the calculation of the overall functional age of users (and/or a related overall index indicative of the state of psycho-physical well-being) by taking into account a plurality of domains and according to a larger data base, providing a more precise and reliable value.

Advantageously, at least one of the local controller 10 and the remote controller 110 is configured to act as a virtual coach that follows and guides each user step by step in performing and/or updating tests, it defines training plans to improve their psycho-physical status and monitors their progress over time.

In particular, the system 100 checks if one or more of the tests has expired and is configured in order to invite the user to perform tests that have not yet been performed or to update those that have expired, so that the assessment of the user and the specific training plan are as up-to-date as possible and in line with the current psycho-physical state of each user.

The system 100 can even be configured to calculate the overall functional age value (and/or said overall index) and define the corresponding training program only when the user has completed all tests and the tests are still temporally valid so as to act as a spur for the user himself or herself to proceed.

Of course, without prejudice to the principle of the invention, the embodiments and details of embodiment may be widely varied with respect to what has been described and illustrated by way of non-limiting example only, without thereby departing from the scope of protection of the present invention as defined by the appended claims. For example, similarly to what has been described for the domain of muscular strength, depending on the specific applications, the remote controller 110 of the system 100 can calculate the functional age (and/or the respective reference index for each of the tested domains, i.e. balance ability, body mobility, cognitive ability, cardiovascular status, body mass composition (muscle mass and/or lean mass), as an average between several values relating to the same domain.

**Claims**

1. A system (100) for assessing and improving the functional age and/or an overall index indicative of a current state of psycho-physical well-being of a user, **characterized by** comprising at least:

   - a check-up station (1) which comprises a local controller (10), at least one of a display (3) and a loudspeaker (5), said check-up station (1) being configured to allow the user to connect to the system (100) either directly on the display (3) itself or by means of an own portable electronic device (125);
   - a remote controller (110) suitable for being placed in communication with at least said check-up station (1) and with one or more pre-selected physical exercise devices (130, 140) whose identifying data is prerecorded in at least one data recording unit (150) accessible from the remote controller (110);
   wherein at least one of said remote (110) and local (10) controllers is configured to present, via at least one of said display (3), portable electronic device (125), and loudspeaker (5), information and/or instructions suitable to guide a user in performing a plurality of tests distinct from each other, each of said tests having a predetermined temporal validity and being related to a physical or psychological domain of the user to be tested, said check-up station (1) being configured to allow the execution of a first subset of tests of the plurality of tests, at least a second subset of tests of the plurality of tests being executable by means of one or more of said physical exercise devices (130, 140);
   and wherein the remote controller (110) is further configured to:

   - calculate, on the basis of received data related to tests performed, a functional age value and/or a reference index specific to each domain whose corresponding test has been performed;
   - calculate an overall functional age value and/or said overall index based on the functional age values and/or reference indexes calculated for the tests performed; and
   - depending on at least one parameter indicative of the tested user, and/or at least one specific functional age value or reference index calculated, and/or the overall functional age value or overall index calculated, generate a new training program or modify/confirm a pre-existing training program making it accessible to the user via at least one of the display (3) and the portable electronic device (125).

2. System (100) according to claim 1, wherein the check-up station (1) comprises said display (3), and wherein said remote controller (110) is further configured to display on at least one of said display (3) and portable electronic device (125) the calculated functional age values and/or reference indexes for each tested domain and the overall functional age value and/or said overall index.

3. System (100) according to claim 1, wherein said remote controller (110) is further configured to:

   - check whether each test of the plurality of tests has been performed and is still temporally valid; and, if not,

- invite the user to execute each test that has not been executed yet and/or repeat a temporally expired test.

4. System (100) according to claim 1 or 2, wherein said remote controller (110) is further configured to calculate an overall functional age value and/or said overall index based on and only when all tests of the plurality of tests to be performed have been performed and are temporally valid.

5. System (100) according to one or more of the preceding claims wherein, in response to the user's selection of a test to be performed belonging to the second subgroup of tests, the remote controller (110) is further configured to:

- send to the user, directly on at least one of said display (3), loudspeaker (5), and said own portable electronic device (125), one or more instructions identifying at least one device among said one or more pre-selected physical exercise devices (130, 140) with which to perform said test belonging to the second test subgroup.

6. System (100) according to the preceding claim, wherein said remote controller (110) is further configured to send on at least one of said at least one identified device and portable electronic device (125) one or more instructions designed to guide the user in performing said test belonging to the second test subgroup.

7. System (100) according to claim 5 or 6, wherein said remote controller (110) is further configured to remotely configure said at least one identified device on which said user has to perform said test belonging to the second test subgroup.

8. System (100) according to one or more of the preceding claims, wherein said check-up station (1) comprises said display (3), wherein at least one of said local controller (10) and remote controller (110) is configured to present, via at least one of said display (3), loudspeaker (5) and said own portable electronic device (125), instructions to the user to assume one or more predetermined positions in front of said display (3), wherein the check-up station (1) includes at least one camera (7) configured to capture one or more first images of the user when the user assumes said one or more predetermined positions, wherein at least one of said local controller (10) and remote controller (110) is configured to calculate, based on the one or more first images captured, one or more anthropometric data of the user.

9. System (100) according to claim 7 or 8, wherein the remote controller (110) is further configured to remotely configure said at least one identified device on which said user has to perform said test belonging to the second test subgroup based on one or more of the anthropometric data calculated for the user or of one or more pre-recorded anthropometric data of the user.

10. System (100) according to one or more of the preceding claims, wherein the check-up station (1) further comprises at least one camera (7) configured to capture one or more second images of the user when the user is performing a first test that belongs to the first test subgroup and is configured to assess a current level of mobility of at least one body part of the user, and when the user is performing a second test that belongs to the first test subgroup and is configured to assess a current level of balance ability of the user; and wherein said remote controller (110) is configured to calculate, on the basis of data processed on the basis of the captured second images and sent by the local controller (10), a specific functional age value and/or a reference index related to the current level of mobility of the user, and a respective specific functional age value and/or a reference index related to the current balance capability of the user.

11. System (100) according to one or more of the preceding claims, wherein at least one of said local controller (10) and remote controller (110) is configured to present, via at least one of said display (3), loudspeaker (5), and said own portable electronic device (125), a plurality of instructions designed to guide the user in performing a test of said plurality of tests that is configured to assess a level of cognitive ability of the user to be performed directly on the check-up station (1) or on a device (115) remote from the check-up station (1) and operatively connectable to the remote controller (110), and wherein the remote controller (110) is further configured to calculate, based on the test performed, a specific functional age value and/or a reference index related to the current level of cognitive capability of the user.

12. System (100) according to one or more of the preceding claims, wherein the check-up station (1) incorporates means (22, 24, 28, 29) for or is connected to a device (20) for measuring at least one parameter indicative of the current body mass composition of said user, wherein at least one of said local controller (10) and said remote controller (110) is configured to present, through at least one of said display (3) and loudspeaker (5), a plurality of instructions designed to guide the user in performing, through said means (22, 24, 28, 29) or said device (20), a test capable of calculating or measuring at least one parameter indicative of the current body mass composition of said user, and wherein said remote controller (110) is further configured to calculate, based on the at least one calculated or measured parameter, a specific functional age value and/or a reference index related to the current body mass composition of the user.

## FIG.1

## FIG.2

## FIG.3

## FIG.4

## FIG.5

## FIG.6

## FIG.7

## FIG.8

## FIG.9

FIG.10

**EUROPEAN SEARCH REPORT**

Europäisches
Patentamt
European
Patent Office
Office européen
des brevets

Application Number

EP 25 16 1255

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 105 574 B2 (INTEL CORP [US]) 23 October 2018 (2018-10-23) | 1-12 | INV. G16H20/40 |
| Y | * abstract; figures 1-4 * * column 1, line 5 - line 20 * * column 2, line 49 - column 3, line 8 * * column 3, line 21 - line 60 * * column 5, line 22 - line 32 * * column 6, line 41 - line 54 * * column 7, line 32 - line 54 * * column 8, line 4 - column 10, line 3 * * column 9, line 26 - line 53 * ----- | 1-12 | G16H50/30 G16H40/63 |
| Y | US 10 052 026 B1 (TRAN BAO [US]) 21 August 2018 (2018-08-21) * abstract; figure 2a * * column 1, line 25 - line 41 * * column 14, line 24 - line 58 * * column 26, line 45 - line 59 * * column 37, line 46 - column 38, line 7 * * column 47, line 8 - column 48, line 39 * ----- | 1-12 | |
| A | US 11 364 418 B2 (PIAZZA JOHN [US]; PIAZZA SUSAN [US]) 21 June 2022 (2022-06-21) * column 1 - column 4 * ----- | 1-12 | **TECHNICAL FIELDS SEARCHED (IPC)** G16H |
| A | US 2014/370479 A1 (GAZZALEY ADAM [US]) 18 December 2014 (2014-12-18) * paragraph [0139] - paragraph [0150] * ----- | 1-12 | |
| A | CN 116 726 457 A (SHENZHEN ZHIQIANKUN TECH CO LTD) 12 September 2023 (2023-09-12) * page 4 - page 5 * ----- | 1-12 | |

The present search report has been drawn up for all claims

| Place of search | Date of completion of the search | Examiner |
|---|---|---|
| Munich | 23 June 2025 | Gardiner, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

......................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 1255

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2025

| Patent document cited in search report | | Publication date | Patent family member(s) | | Publication date |
|---|---|---|---|---|---|
| US 10105574 | B2 | 23-10-2018 | NONE | | |
| US 10052026 | B1 | 21-08-2018 | US | 10052026 B1 | 21-08-2018 |
| | | | US | 2018253840 A1 | 06-09-2018 |
| US 11364418 | B2 | 21-06-2022 | NONE | | |
| US 2014370479 | A1 | 18-12-2014 | AU | 2011326372 A1 | 30-05-2013 |
| | | | AU | 2017200417 A1 | 09-02-2017 |
| | | | AU | 2018260916 A1 | 29-11-2018 |
| | | | AU | 2020213392 A1 | 27-08-2020 |
| | | | AU | 2022201778 A1 | 07-04-2022 |
| | | | AU | 2024202412 A1 | 02-05-2024 |
| | | | CA | 2720892 A1 | 12-05-2012 |
| | | | CA | 2816910 A1 | 18-05-2012 |
| | | | CA | 3085039 A1 | 18-05-2012 |
| | | | EP | 2638534 A1 | 18-09-2013 |
| | | | EP | 3863000 A1 | 11-08-2021 |
| | | | JP | 6177131 B2 | 09-08-2017 |
| | | | JP | 6297096 B2 | 20-03-2018 |
| | | | JP | 6470338 B2 | 13-02-2019 |
| | | | JP | 6666938 B2 | 18-03-2020 |
| | | | JP | 7049379 B2 | 06-04-2022 |
| | | | JP | 7431272 B2 | 14-02-2024 |
| | | | JP | 2014508309 A | 03-04-2014 |
| | | | JP | 2016179193 A | 13-10-2016 |
| | | | JP | 2017161910 A | 14-09-2017 |
| | | | JP | 2018141964 A | 13-09-2018 |
| | | | JP | 2020106853 A | 09-07-2020 |
| | | | JP | 2022091882 A | 21-06-2022 |
| | | | JP | 2024045380 A | 02-04-2024 |
| | | | US | 2014370479 A1 | 18-12-2014 |
| | | | US | 2018261115 A1 | 13-09-2018 |
| | | | US | 2022036752 A1 | 03-02-2022 |
| | | | US | 2025174141 A1 | 29-05-2025 |
| | | | WO | 2012064999 A1 | 18-05-2012 |
| CN 116726457 | A | 12-09-2023 | NONE | | |

EPO FORM P0459

For more details about this annex : see Official Journal of the European Patent Office, No. 12/82